(19) Europäisches Patentamt
European Patent Office
Office européen des brevets

(11) Veröffentlichungsnummer : **0 048 368**
**B1**

(12) # EUROPÄISCHE PATENTSCHRIFT

(45) Veröffentlichungstag der Patentschrift : **13.06.84**

(21) Anmeldenummer : **81106989.7**

(22) Anmeldetag : **05.09.81**

(51) Int. Cl.$^3$ : **C 07 C125/065**, C 07 C125/073, C 07 C118/00

(54) **Verfahren zur Herstellung von N- und O-substituierten Mono- oder Bis-urethanen.**

(30) Priorität : **18.09.80 DE 3035146**

(43) Veröffentlichungstag der Anmeldung : **31.03.82 Patentblatt 82/13**

(45) Bekanntmachung des Hinweises auf die Patenterteilung : **13.06.84 Patentblatt 84/24**

(84) Benannte Vertragsstaaten : **BE CH DE FR GB IT LI NL**

(56) Entgegenhaltungen :
**DE-A- 1 944 719**
**DE-A- 2 530 001**
**GB-A- 1 231 946**
**US-A- 3 265 728**

(73) Patentinhaber : **BAYER AG**
**Konzernverwaltung RP Patentabteilung**
**D-5090 Leverkusen 1 Bayerwerk (DE)**

(72) Erfinder : **Krimm, Heinrich, Dr.**
**Heyenbaumstrasse 65**
**D-4150 Krefeld (DE)**
Erfinder : **Buysch, Hans-Josef, Dr.**
**Brandenburger Strasse 28**
**D-4150 Krefeld (DE)**

**EP 0 048 368 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neuartiges Verfahren zur Herstellung von am Stickstoff und Sauerstoff aliphatisch substituierten Mono- und Bis-urethanen durch Umesterung der entsprechenden O-(2-Hydroxyalkyl)-substituierten Urethane mit hochsiedenden, 1-wertigen primären Alkoholen oder mit 2-wertigen aliphatischen Alkoholen mit primären Hydroxylgruppen die nicht in 2-Stellung zueinander angeordnet sind.

Primäre Amine mit aliphatisch oder cycloaliphatisch gebundenen Aminogruppen reagieren bekanntlich mit Glykolcarbonat in praktisch quantitativer Ausbeute unter Bildung der entsprechenden N-substituierten Carbamidsäure-β-hydroxyethylester nach folgender Gleichung :

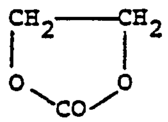 $+ R{-}NH_2 \longrightarrow R{-}NH{-}CO{-}O{-}CH_2{-}CH_2{-}OH$

Das hierbei zum Einsatz gelangende Ethylenglykolcarbonat, das bei der Umsetzung auch beispielsweise durch Propylenglykolcarbonat ersetzt werden kann, ist seinerseits auf einfache, an sich bekannter Weise gemäß

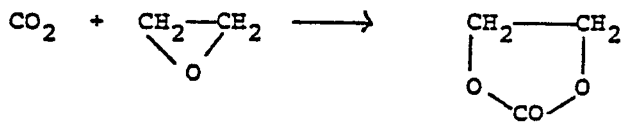

aus wohlfeilen, großtechnischen Ausgangsmaterialien (Kohlendioxid und Ethylenoxid (oder Propylenoxid)) zugänglich. Es ist demnach möglich, aus den genannten einfachen, großtechnischen Ausgangsmaterialien je nach Art des eingesetzten primären Amins die unterschiedlichsten N-substituierten Carbamidsäure-β-hydroxyalkylester herzustellen. Der Gedanke, diese Reaktionsschritte zur phosgenfreien Herstellung von organischen Isocyanaten, insbesondere mit aliphatisch gebundenen Isocyanatgruppen zu nutzen, scheitert an dem Umstand, daß die genannten Carbamidsäure-β-hydroxyalkylester nicht thermisch in das ihnen zugrundeliegende Isocyanat und das ihnen zugrundeliegende Glykol gespalten werden können.

Es war daher die Aufgabe der vorliegenden Erfindung, ein Verfahren zur Verfügung zu stellen, welches die Überführung der nicht in Isocyanat und Glykol spaltbaren Carbamidsäure-β-hydroxyalkylester in solche Carbamidsäureester gestattet, die einer thermischen Spaltung unter Bildung des ihnen zugrundeliegenden Isocyanats zugänglich sind.

Diese Aufgabe konnte dadurch gelöst werden, daß die genannten Carbamidsäure-β-hydroxyalkylester in Gegenwart von ausgewählten Katalysatoren mit bestimmten, nachstehend näher beschriebenen Alkoholen unter Freisetzung der den Carbamidsäureestern zugrundeliegenden Glykole umgeestert werden.

Die Umesterung von Carbamidsäureestern mit höheren Alkoholen ist bereits bekannt. Gemäß DE-PS 565 319 erfolgt die Umsetzung im Temperaturbereich von 190-230 °C unter Verwendung von Natriummetall bzw. -alkoholat als Katalysator. Gemäß US-PS 2 934 559 können Biscarbamidsäureester aus Carbamidsäureestern und Alkandiolen unter Verwendung von Aluminiumalkoholaten als Katalysatoren hergestellt werden. Schließlich offenbart die DE-OS 2 655 741 die Herstellung von Carbamidsäureestern auf Basis höhersiedender Alkohole durch Umesterung von Carbamidsäureestern auf Basis von niedrigsiedenden Alkoholen mit höhersiedenden Alkoholen in Gegenwart von Titanalkoholaten als Katalysatoren.

Bei allen diesen Verfahren des Standes der Technik werden Carbamidsäureester, d. h. Urethane eingesetzt, die am Stickstoff unsubstituiert sind. Offenbar ist die Umesterung von N-substituierten Carbamidsäure-β-hydroxyphenylestern mit den bekannten Verfahren nicht zu vergleichen. Das ist einerseits mit der Substitution am Stickstoff zu erklären, andererseits mit deren größerer Empfindlichkeit gegenüber thermischen Abbau, da bei Temperaturen oberhalb 60 °C leicht Kohlendioxid bzw. Wasser abgespalten und Amine bzw. Oxazolinone gebildet werden. So nimmt es nicht wunder, daß die bisher vorgeschlagenen Katalysatoren entweder unwirksam sind (Aluminiumalkoholate) oder einen glatten

Reaktionsverlauf, d. h. optimale Ausbeuten nicht gewährleisten (Natriumalkoholat oder Titantetrabutylat).

Wie nun überraschend gefunden wurde, gelingt auch die Umesterung von N-substituierten Carbamidsäure-β-hydroxyalkylestern mit höhersiedenden Alkoholen unter Freisetzung der den Ausgangsmaterialien zugrundeliegenden Glykole, wenn die nachstehend näher beschriebenen erfindungswesentlichen Katalysatoren eingesetzt werden.

Gegenstand der vorliegenden Erfindung ist somit ein Verfahren zur Herstellung von N- und O-substituierten Mono- oder Bis-urethanen, dadurch gekennzeichnet, daß man Urethane der Formel

$$R^1\text{—}(NH\text{—}CO\text{—}O\text{—}R^2)_n$$

in welcher

$R^1$ für einen n-wertigen, aliphatischen, gegebenenfalls Etherbrücken aufweisenden aliphatischen Kohlenwasserstoffrest mit 1 bis 6 Kohlenstoffatomen oder einen n-wertigen cycloaliphatischen Kohlenwasserstoffrest mit 6 bis 13 Kohlenstoffatomen steht,

$R^2$ für eine Hydroxyalkylgruppe mit 2 bis 3 Kohlenstoffatomen steht, wobei zwischen dem Sauerstoffatom der Hydroxylgruppe und dem Sauerstoffatom der Urethangruppe 2 Kohlenstoffatome angeordnet sind, und

n für 1 oder 2 steht,

mit ausschließlich primäre Hydroxylgruppen aufweisenden Alkoholen der Formel

$$R^3\text{—}(OH)_m$$

in welcher

$R^3$ für einen einwertigen (m = 1), linearen oder verzweigten, gesättigten, gegebenenfalls Etherbrücken aufweisenden aliphatischen Kohlenwasserstoffrest mit 8 bis 18 Kohlenstoffatomen, einen einwertigen (m = 1), araliphatischen Kohlenwasserstoffrest mit 8 bis 15 Kohlenstoffatomen oder einen zweiwertigen (m = 2), linearen oder verzweigten, gesättigten, gegebenenfalls Etherbrücken aufweisenden aliphatischen Kohlenwasserstoffrest mit 3-18 Kohlenstoffatomen steht, in dem zwischen den beiden Hydroxylgruppen mindestens 3 Kohlenstoffatome angeordnet sind, und

m für 1 oder 2 steht, mit der Maßgabe daß m und n nicht gleichzeitig 2 bedeuten können,

in Gegenwart von Katalysatoren ausgewählt aus der Gruppe bestehend aus (i) Oxiden, Hydroxiden, Salzen organischer Säuren, Salzen schwacher anorganischer Säuren und organischen Komplexverbindungen des 1-wertigen Thalliums, sowie (ii) Salzen organischer Säuren des 2- und 4-wertigen Zinns und organischen Verbindungen des 4-wertigen Zinns mit nicht salzartig gebundenen organischen Resten, unter Abspaltung von Alkoholen der Formel

$$R^2\text{—}OH$$

einer Umesterungsreaktion unterzieht.

Ausgangsmaterialien für das erfindungsgemäße Verfahren sind Carbamidsäureester der Formel

$$R^1\text{—}(NH\text{—}CO\text{—}O\text{—}R^2)_n$$

und Alkohole mit ausschließlich primären Hydroxylgruppen der Formel

$$R^3\text{—}(OH)_m .$$

In diesen Formeln haben $R^1$, $R^2$, $R^3$, m und n die bereits obengenannte Bedeutung. Vorzugsweise stehen

$R^1$ für einen gesättigten, unsubstituierten, aliphatischen Kohlenwasserstoffrest mit 1 bis 6 Kohlenstoffatomen,

$R^2$ für einen 2-Hydroxypropyl- oder einen Hydroxyethyl-Rest, insbesondere für einen Hydroxyethyl-Rest,

$R^3$ für einen gesättigten, unsubstituierten, aliphatischen Kohlenwasserstoffrest mit 8 bis 18, insbesondere 10 bis 14 Kohlenstoffatomen und

m und n jeweils für 1.

Diese Definition der Variablen gilt auch nachstehend.

Die beim erfindungsgemäßen Verfahren einzusetzenden N-substituierten Carbamidsäure-β-hydroxyalkylester werden, wie bereits ausgeführt, in an sich bekannter Weise durch Umsetzung der ihnen entsprechenden Glykolcarbonate mit den ihn entsprechenden Aminen erhalten.

Hierzu geeignete Glykolcarbonate sind insbesondere Propylenglykolcarbonat und Ethylenglykolcarbonat. Ethylenglykolcarbonat ist das bevorzugte Ausgangsmaterial. Die Glykolcarbonate entstehen ihrerseits durch Umsetzung der ihnen zugrundeliegenden Alkylenoxide mit Kohlendioxid.

Geeignete Amine sind beliebige Verbindungen der Formel

$R^1—(NH_2)_n$.

Typische Beispiele geeigneter Amine sind Methylamin, Ethylamin, n-Propylamin, Isopropylamin, n-Butylamin, Isobutylamin, n-Pentylamin, n-Hexylamin, Cyclohexylamin, Ethylendiamin, Tetramethylendiamin, Hexamethylendiamin, 4,4'-Diaminodicyclohexylmethan oder Isophorondiamin.

Für das erfindungsgemäße Verfahren sind im Prinzip alle 1- und 2-wertigen Alkohole geeignet, die ausschließlich primäre Hydroxylgruppen aufweisen, in denen keine β-ständig zueinander angeordnete Hydroxylgruppen vorliegen und die bei Normaldruck einen Siedepunkt aufweisen, der höchstend 10 °C unter dem Siedepunkt des bei der erfindungsgemäßen Umsetzung sich bildenden Glykols liegt. Vorzugsweise werden solche Alkohole eingesetzt, deren Siedepunkt bei Normaldruck mindestens so hoch liegt wie der Siedepunkt des abzuspaltenden Glykols. Besonders bevorzugt sind indessen solche Alkohole, deren Siedepunkt bei Normaldruck mindestens 10 °C über dem Siedepunkt des abzuspaltenden Glykols liegt. Typische Beispiele geeigneter Alkohole sind Octanol, Nonanol, Decanol, Undecanol, Dodecanol oder Stearylalkohol mit jeweils linearer oder verzweigter Kohlenstoffkette, 2-Phenyl-ethanol, ferner 2-wertige Alkohole wie Butandiol-1,4 oder Hexandiol-1,6. Im Prinzip können selbstverständlich auch Ethergruppen aufweisende Alkohole, wie beispielsweise Ethoxylierungsprodukte von einfachen Alkoholen wie Methanol, Ethanol oder Ethylenglykol eingesetzt werden, soweit die obengenannten Bedingungen bezüglich des Siedepunkts eingehalten werden. Auch der Einsatz von mehr als 2-wertigen Alkoholen wie beispielsweise von Trimethylolpropan ist im Prinzip möglich.

Für das erfindungsgemäße Verfahren geeignete Katalysatoren sind beispielsweise Dibutylzinnoxid, Dibutylzinndilaurat, Dimethoxydibutylzinn, Ethylzinntriisooctylat, Zinndilaurat und Zinnacetat, ferner Thalliumhydroxid, Thalliumoxid, Thalliumcarbonat, Thalliumacetat, Thalliumoctoat, Thalliumnaphthenat, Thalliumbenzoat oder organische Komplexverbindungen des 1-wertigen Thalliums, beispielsweise solchen auf Basis von Cyclopentadien und löslichen Thalliumsalzen.

Die Katalysatoren werden in Mengen von 0,001-1 %, vorzugsweise 0,01-0,5 %, bezogen auf das Reaktionsgemisch, eingesetzt.

Die Reaktionstemperaturen liegen im Bereich von 100-180 °C, vorzugsweise von 120-150 °C.

Im Interesse einer möglichst quantitativen Umesterung ist es zweckmäßig, die Alkohole in mindestens äquivalenten Mengen, bezogen auf Urethangruppen des umzuesternden Carbamidsäureesters, einzusetzen. Vorzugsweise wird eine 1,2-10-fach, insbesondere eine 1,5-5-fach äquivalente Menge des Alkohols verwendet. Im Falle der Verwendung von mehrwertigen Alkoholen ist es allerdings zweckmäßig lediglich unter Verwendung von äquivalenten Mengen der Reaktionspartner zu arbeiten, damit hydroxylgruppenfreie Verfahrensprodukte entstehen. Die Abtrennung des sich während der erfindungsgemäßen Umsetzung bildenden Glykols kann durch gleichzeitige Mitverwendung von Schleppmitteln wie Xylol, Ethylbenzol oder Cumol erleichtert werden.

Zur Durchführung des erfindungsgemäßen Verfahrens werden die Reaktionspartner zusammen mit dem Katalysator vorzugsweise unter Vakuum auf die Reaktionstemperatur erhitzt und das sich bildende Glykol $R^2$-OH beispielsweise über eine Destillationskolonne fortlaufend abdestilliert. Bei Verwendung von Alkoholen mit einem Siedepunkt, der dem Siedepunkt des Glykols entspricht bzw. der bis zu 10 °C unter dem Siedepunkt des Glykols liegt, kann die Abtrennung des Glykols im Gemisch mit gleichzeitig destillierendem, im Überschuß vorliegendem Alkohol erfolgen.

Falls die Reaktionsprodukte der Umesterung destilliert werden sollen, ist es vielfach zweckmäßig, den Umesterungskatalysator durch Behandlung mit einem Ionentauscher wie einer saureaktivierten Bleicherde oder einem sulfonierten vernetzten Polystyrol zu entfernen, um Zersetzungen zu vermeiden.

Die bei der erfindungsgemäßen Umsetzung als Rückstand anfallenden Urethane der Formel

$$R^1—(NH—CO—O—R^3)_n$$

bzw.

$$(R^1—NH—CO—O)_m—R^3$$

können nach Entfernung des evtl. noch vorliegenden überschüssigen Alkohols oder nach ihrer destillativen Reindarstellung in an sich bekannter Weise thermisch in die ihnen zugrundeliegenden Isocyanate der Formel

$$R^1—(NCO)_n$$

und die ihnen zugrundeliegenden Alkohole der Formel

$$R^3—(OH)_m$$

zerlegt werden. Die thermische Spaltung kann auch unter Mitverwendung von geeigneten Katalysatoren bewirkt werden. Als Katalysatoren sind Feststoffe wie Aluminiumoxid, Siliciumdioxid, Kieselgur, Kaolin,

Talkum, Aluminiumpulver, Eisenpulver sowie Bimsstein, der mit geringen Mengen von Substanzen wie Antimontrioxid, Eisenoxid oder Vanadinoxid imprägniert ist, brauchbar.

Zweckmäßig werden die Urethane durch eine heiße Reaktionszone geführt, wobei in Abhängigkeit von der Temperatur und der Verweilzeit eine gewisse Spaltungsrate erzielt wird. Die Isocyanate und die höheren Alkohole werden durch Destillation vom unumgesetzten Ausgangsmaterial getrennt, das wieder in den Spaltprozeß zurückgeführt wird.

Die Spaltungstemperaturen liegen im allgemeinen bei 280-350 °C.

Die in den nachfolgenden Beispielen angegebenen Prozentangaben beziehen sich auf Gewichtsprozente.

Herstellung der N-Alkyl-carbamidsäure-β-hydroxyethylester

Beispiel 1

In 880 g (10 Mol) Glykolcarbonat läßt man unter Kühlung bei 35-70 °C 780 g einer 40 %igen wäßrigen Methylaminlösung (10 Mol) eintropfen. Man entfernt das Wasser bei 90 °C/20 Torr und destilliert den Rückstand. Bei 104-106°/0,06 Torr gehen 1 153 g (= 97 % d. Th.) N-Methyl-carbamidsäure-β-hydroxyethylester über.

Beispiel 2

In 176 g (2 Mol) Glykolcarbonat läßt man bei 70-80 °C unter Kühlung 116 g (1 Mol) Hexamethylendiamin eintropfen. Der N,N'-Hexamethylen-bis-carbamidsäure-β-hydroxyethylester kristallisiert sofort aus. Schmelzpunkt : 89-90 °C.

Ausbeute : quantitativ.

Beispiel 3

In 88 g (1 Mol) Glykolcarbonat läßt man bei 70-100 °C 99 g (1 Mol) Cyclohexylamin eintropfen. Der N-Cyclohexyl-carbamidsäure-β-hydroxyethylester erstarrt kristallin beim Erkalten.

Schmp. : 65-66 °C. Ausbeute : quantitativ.

Herstellung der N-Alkyl-carbamidsäure-alkylester

Beispiel 4

119 g (1 Mol) N-Methyl-β-hydroxyethylurethan, 270 g (1,5 Mol) Dodecanol-1 und 1 g Dimethoxydibutylzinn werden an einer 40 cm-Füllkörperkolonne unter 12 Torr auf 140°C erhitzt, während über Kopf bei 100-103 °C ein zweiphasiges Gemisch von Glykol und etwas Dodecanol übergeht. Nach 6 Stunden sind 66 g Destillat mit etwa der berechneten Menge Glykol abgetrennt. Man destilliert das restliche überschüssige Dodecanol bei 60-74 °C/0,1 Torr ab (87 g) und erhält 242 g Rückstand. Aus dessen Lösung in Ligroin fallen 235 g Kristalle vom Schmp. 54 °C aus. Ausbeute an N-Methyldodecylurethan, 97 % d. Th.

Beispiel 5

Der Ansatz gemäß Beispiel 4 wird mit dem Unterschied wiederholt, daß anstelle von Dimethoxydibutylzinn 0,5 g Ethylzinntriisooctylat eingesetzt wird. Nach 4 1/2 Stunden sind bei einer Reaktionstemperatur von 130 °C 65 g Destillat abgetrennt. Nach dem Abdestillieren des überschüssigen Dodecanols werden 238 g Rohprodukt erhalten. Hieraus gewinnt man 231 g (= 95 % d. Th.) an Reinprodukt durch Destillation.

Beispiel 6

Beispiel 4 wird wiederholt. Als Katalysator wird 0,1 g Thalliumcarbonat eingesetzt. Die Reaktionstemperatur ist 140 °C. Die Reaktionszeit 7 Stunden. Das Destillat beträgt 71 g, Rohprodukt 204 g. Zur Entfernung des Katalysators wird dieses mit 10 g säureaktivierter Bleicherde bei 100 °C behandelt, filtriert und destilliert. Bei 112°/0,06 Torr gehen 175 g (72 % d. Th.) Reinprodukt über.

Beispiel 7

119 g (1 Mol) N-Methyl-β-hydroxyethylurethan, 316 g (2 Mol) Decanol-1 und 0,1 g Thalliumcarbonat werden gemäß Beispiel 4 umgesetzt. Nach 4 Stunden sind 125 g Destillat abgetrennt. Gemäß fraktionierter Destillation sind darin 62 g Glykol und 63 g Decanol enthalten. Der Rückstand nach dem Abdestillieren des restlichen Decanols beträgt 216 g. Man entfernt den Katalysator wie in Beispiel 6 und

destilliert das Reaktionsprodukt. Man erhält bei 95-97°/0,05 Torr 192 g (= 88 % d. Th.) N-Methyl-decylurethan. Schmp. : 46 °C.

Beispiel 8

146 g (0,5 Mol) N,N'-Hexamethylen-bis-β-hydroxyethylurethan, 465 g (2,5 Mol) Dodecanol und 100 mg Thalliumoxid werden wie in Beispiel 4 umgesetzt. Nach 5 Stunden sind bei 90-95 °C/8 Torr 76 g Destillat abgetrennt. Man behandelt den Rückstand mit 20 g säureaktivierter Bleicherde und destilliert aus dem katalysatorfreien Reaktionsprodukt das nicht umgesetzte Dodecanol ab (259 g). Das kristallisierte N,N'-Hexamethylen-bis-dodecylurethan bleibt als Rückstand. Schmp. 111-112 °C. Ausbeute 266 g = 98 % d. Th.

Beispiel 9

72 g (0,2 Mol) Dicyclohexylmethan-bis-β-hexylethylurethan, hergestellt aus dem 4,4'-Diaminodicyclohexylmethan-Isomerengemisch, 167 g (0,9 Mol) Dodecanol und 0,5 g Dibutylzinndilaurat werden wie in Beispiel 4 umgesetzt. Nach 5 Stunden sind 28 g Destillat, vorwiegend Glykol, abgetrennt. Nach Abdestillieren des überschüssigen Dodecanols bei 77-80 °C/0,04 Torr bleiben 120 g Rückstand. Ausbeute an N,N'-Methylendicyclohexyl-bis-dodecylurethan 98 % d. Th. Schmp. (aus Methanol) 116-127 °C.

Beispiel 10

59,5 g (0,5 Mol) N-Methyl-β-hydroxyethylurethan, 118 g (1 Mol) Hexandiol-1,6 und 2 g Dibutylzinnoxid werden an einer 40 cm-Füllkörperkolonne auf 125-140 °C/0,1 Torr gehalten. Über Kopf sind nach 4 Stunden 32 g Glykol abdestilliert. Aus dem Reaktionsprodukt wird das überschüssige Hexandiol bei 88-92 °C/0,04 Torr entfernt. Man erhält 56 g Rückstand. Das N,N'-Dimethyl-hexamethylen-bisurethan wird aus Toluol umkristallisiert. Schmp. 124-126 °C.

Herstellung von Isocyanaten durch Spaltung von Urethanen

Beispiel 11

Man läßt im Laufe von 1 1/2 Stunden durch einen Tropftrichter 30 g N-Methylcarbamidsäuredodecylester in einen mit 5 g Kieselgur beschickten, mit einem Metallbad auf 300 °C vorgeheizten Kolben tropfen, während die bei 210-220 °C übergehenden Spaltprodukte in einer eisgekühlten, mit Chlorbenzol beschickten Vorlage aufgefangen werden. Durch Titration mit Dibutylamin wird der Gehalt an Methylisocyanat bestimmt : 6,4 g = 90 % d. Th.

Beispiel 12

Man läßt im Laufe von 2 Stunden 54 g (0,1 Mol) N,N'-Hexamethylen-bis-carbamidsäuredodecylester in einen auf 320 °C vorgeheizten Kolben unter 150 Torr eintropfen, während die Spaltprodukte bei 220-240 °C übergehen und in einer eisgekühlten Vorlage aufgefangen werden. Gemäß Titration mit Dibutylamin sind im Destillat 13,5 g (= 80 % d. Th.) an Hexamethylendiisocyanat enthalten.

**Ansprüche**

1. Verfahren zur Herstellung von N- und O-substituierten Mono- oder Bis-urethanen, dadurch gekennzeichnet, daß man Urethane der Formel

$$R^1—(NH—CO—O—R^2)_n$$

in welcher

$R^1$ für einen n-wertigen, aliphatischen, gegebenenfalls Etherbrücken aufweisenden aliphatischen Kohlenwasserstoffrest mit 1 bis 6 Kohlenstoffatomen oder einen n-wertigen cycloaliphatischen Kohlenwasserstoffrest mit 6 bis 13 Kohlenstoffatomen steht,

$R^2$ für eine Hydroxyalkylgruppe mit 2 bis 3 Kohlenstoffatomen steht, wobei zwischen dem Sauerstoffatom der Hydroxylgruppe und dem Sauerstoffatom der Urethangruppe 2 Kohlenstoffatome angeordnet sind, und

n für 1 oder 2 steht,

mit ausschließlich primäre Hydroxylgruppen aufweisenden Alkoholen der Formel

$$R^3—(OH)_m$$

in welcher

$R^3$ für einen einwertigen (m = 1), linearen oder verzweigten, gesättigten, gegebenenfalls Etherbrücken aufweisenden, aliphatischen Kohlenwasserstoffrest mit 8 bis 18 Kohlenstoffatomen, einen einwertigen (m = 1), araliphatischen Kohlenwasserstoffrest mit 8 bis 15 Kohlenstoffatomen oder einen zweiwertigen (m = 2), linearen oder verzweigten, gesättigten, gegebenenfalls Etherbrücken aufweisenden, aliphatischen Kohlenwasserstoffrest mit 3-18 Kohlenstoffatomen steht, in dem zwischen den beiden Hydroxylgruppen mindestens 3 Kohlenstoffatome angeordnet sind, und

m für 1 oder 2 steht, mit der Maßgabe daß m und n nicht gleichzeitig 2 bedeuten können,

in Gegenwart von Katalysatoren ausgewählt aus der Gruppe bestehend aus (i) Oxiden, Hydroxiden, Salzen organischer Säuren, Salzen schwacher anorganischer Säuren und organischen Komplexverbindungen des 1-wertigen Thalliums, sowie (ii) Salzen organischer Säuren des 2- und 4-wertigen Zinns und organischen Verbindungen des 4-wertigen Zinns mit nicht salzartig gebundenen organischen Resten, unter Abspaltung von Alkoholen der Formel

$$R^2—OH$$

einer Umesterungsreaktion unterzieht.

2. Verfahren gemäß Anspruch 1, dadurch gekennzeichnet, daß man als Verbindungen der Formel

$$R^1—(NH—CO—O—R^2)_n$$

solche verwendet, für welche

$R^1$ für einen 1-wertigen aliphatischen Kohlenwasserstoffrest mit 1 bis 6 Kohlenstoffatomen steht,

$R^2$ die in Anspruch 1 genannte Bedeutung hat und

n 1 bedeutet

und man die Umesterungsreaktion mit solchen Alkoholen der Formel

$$R^3—(OH)_m$$

durchführt, für welche

$R^3$ für einen 1-wertigen aliphatischen Kohlenwasserstoffrest mit 8 bis 18 Kohlenstoffatomen und m 1 bedeutet.

3. Verfahren gemäß Anspruch 1 und 2, dadurch gekennzeichnet, daß man solche Verbindungen der Formel

$$R^1—(NH—CO—O—R^2)_n$$

verwendet, für welche

$R^2$ für einen Hydroxyethylrest steht.

**Claims**

1. A process for the production of N- and O-substituted mono- or bis-urethanes, characterized in that urethanes corresponding to the following formula

$$R^1—(NH—CO—O—R^2)_n$$

in which

$R^1$ represents an n-functional, aliphatic $C_1$-$C_6$-hydrocarbon radical optionally containing ether bridges or an n-functional cycloaliphatic $C_6$-$C_{13}$-hydrocarbon radical,

$R^2$ represents a hydroxyalkyl group containing from 2 to 3 carbon atoms, 2 carbon atoms being present between the oxygen atom of the hydroxyl group and the oxygen atom of the urethane group, and

n = 1 or 2,

are subjected to a transesterification reaction with alcohols containing only primary hydroxyl groups and corresponding to the following formula

$$R^3—(OH)_m$$

in which

$R^3$ represents a monofunctional (m = 1), linear or branched, saturated aliphatic $C_8$-$C_{18}$-hydrocarbon radical optionally containing ether bridges, a monofunctional (m = 1) araliphatic $C_8$-$C_{15}$-hydrocarbon radical or a difunctional (m = 2), linear or branched, saturated aliphatic $C_3$-$C_{18}$-hydrocarbon radical optionally containing ether bridges in which at least 3 carbon atoms are present between the two hydroxyl groups, and

m = 1 or 2 with the proviso that m and n cannot both have the value 2,

in the presence of catalysts selected from the group comprising (i) oxides, hydroxides, salts of organic acids, salts of weak organic acids and organic complex compounds of monovalent thallium, and (ii) organic acid salts of 2- and 4-valent tin and organic compounds of 4-valent tin containing organic radicals which are not bound like salts, with elimination of alcohols corresponding to the following formula

$$R^2—OH.$$

2. A process as claimed in Claim 1, characterized in that the compounds corresponding to the formula

$$R^1—(NH—CO—O—R^2)_n$$

used are those in which
$R^1$ represents a monofunctional aliphatic hydrocarbon radical containing from 1 to 6 carbon atoms,
$R^2$ is as defined in Claim 1 and
$n = 1$
and the transesterification reaction is carried out with alcohols corresponding to the formula

$$R^3—(OH)_m$$

in which
$R^3$ represents a monofunctional aliphatic hydrocarbon radical containing from 8 to 18 carbon atoms and
$m = 1$.

3. A process as claimed in Claims 1 and 2, characterized in that the compounds corresponding to the formula

$$R^1—(NH—CO—O—R^2)_n$$

used are those in which
$R^2$ represents a hydroxyethyl radical.

**Revendications**

1. Procédé de production de mono- ou de bis-uréthannes N- et O-substitués, caractérisé en ce qu'on soumet à une réaction de transestérification des uréthannes de formule

$$R^1—(NH—CO—O—R^2)_n$$

dans laquelle
$R^1$ désigne un reste d'hydrocarbure aliphatique n-valent présentant éventuellement des ponts éther, ayant 1 à 6 atomes de carbone ou un reste d'hydrocarbure cycloaliphatique n-valent ayant 6 à 13 atomes de carbone,
$R^2$ désigne un groupe hydroxyalkyle ayant 2 ou 3 atomes de carbone, avec 2 atomes de carbone disposés entre l'atome d'oxygène du groupe hydroxyle et l'atome d'oxygène du groupe uréthanne, et
n est égal à 1 ou à 2,
avec des alcools portant des groupes hydroxyle exclusivement primaires de formule

$$R^3—(OH)_m$$

dans laquelle
$R^3$ désigne un reste d'hydrocarbure aliphatique monovalent ($m = 1$), linéaire ou ramifié, saturé, portant éventuellement des ponts éther, ayant 8 à 18 atomes de carbone, un reste d'hydrocarbure araliphatique monovalent ($m = 1$) ayant 8 à 15 atomes de carbone ou un reste d'hydrocarbure aliphatique divalent ($m = 2$) saturé, linéaire ou ramifié, portant éventuellement des ponts éther, ayant 3 à 18 atomes de carbone, dans lequel au moins 3 atomes de carbone sont disposés entre les deux groupes hydroxyle, et est égal à 1 ou 2, sous réserve que m et n ne puissent pas en même temps être égaux à 2, en présence de catalyseurs choisis dans le groupe comprenant (i) des oxydes, des hydroxydes, des sels d'acides organiques, des sels d'acides inorganiques faibles et des composés organiques complexes du thallium monovalent, ainsi que (ii) des sels d'acides organiques de l'étain divalent et tétravalent et des composés organiques de l'étain tétravalent avec des restes organiques non liés à la manière d'un sel, avec élimination d'alcools de formule

$$R^2—OH.$$

2. Procédé suivant la revendication 1, caractérisé en ce qu'on utilise comme composés de formule

$$R^1\text{—}(NH\text{—}CO\text{—}O\text{—}R^2)_n$$

des composés pour lesquels
$R^1$ désigne un reste d'hydrocarbure aliphatique monovalent ayant 1 à 6 atomes de carbone,
$R^2$ a la définition donnée dans la revendication 1, et
n est égal à 1
et on conduit la réaction de transestérification avec des alcools de formule

$$R^3\text{—}(OH)_m$$

dans laquelle
$R^3$ désigne un reste d'hydrocarbure aliphatique monovalent ayant 8 à 18 atomes de carbone et
m est égal à 1.

3. Procédé suivant les revendications 1 et 2, caractérisé en ce qu'on utilise des composés de formule

$$R^1\text{—}(NH\text{—}CO\text{—}O\text{—}R^2)_n$$

dans laquelle
$R^2$ désigne un reste hydroxyéthyle.